(19) Europäisches Patentamt
European Patent Office
Office européen des brevets

(11) Numéro de publication : **0 395 672 B1**

(12) # FASCICULE DE BREVET EUROPEEN

(45) Date de publication du fascicule du brevet : **30.12.92 Bulletin 92/53**

(51) Int. Cl.$^5$ : **A61K 31/70**

(21) Numéro de dépôt : **88909539.4**

(22) Date de dépôt : **13.10.88**

(86) Numéro de dépôt international : **PCT/FR88/00506**

(87) Numéro de publication internationale : **WO 89/03217 20.04.89 Gazette 89/09**

(54) **APPLICATION ANTIVIRALE DES OLIGONUCLEOTIDES ALPHA.**

(30) Priorité : **14.10.87 FR 8714200**

(43) Date de publication de la demande : **07.11.90 Bulletin 90/45**

(45) Mention de la délivrance du brevet : **30.12.92 Bulletin 92/53**

(84) Etats contractants désignés : **AT BE CH DE FR GB IT LI LU NL SE**

(56) Documents cités :
**DE-A- 3 125 291**
**FR-A- 2 607 507**
**EXPERIENTIA, vol. 29, nr. 9, 15 September 1973; U.SEQUIN, pp. 1059-1062**
**DIALOG INFORMATION SERVICES, Data Base 155: MEDLINE, accession number 85254502 & Cancer Res., vol. 45, supplement 9,September 1985; pp. 4677-4684**

(73) Titulaire : **CENTRE NATIONAL DE LA RECHERCHE SCIENTIFIQUE**
**15, quai Anatole France**
**F-75007 Paris (FR)**

(72) Inventeur : **IMBACH, Jean-Louis**
**Chemin Clos-des-Oliviers 1108, rue de Las-Sorbes**
**F-34000 Montpellier (FR)**
Inventeur : **RAYNER, Bernard**
**Chênes Colombière, G 68 180, avenue d'Occitanie**
**F-34100 Montpellier (FR)**
Inventeur : **DE CLERQ, Erik**
**35, Paul Lebrunstraat Boîte 7**
**B-3000 Leuven (BE)**

(74) Mandataire : **Warcoin, Jacques et al**
**Cabinet Régimbeau 26, avenue Kléber**
**F-75116 Paris (FR)**

EP 0 395 672 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention se rapporte, d'une manière générale, aux composés chimiques constitués par un oligonucléotide ou un oligodésoxynucléotide comportant un enchaînement de nucléotides possédant la configuration anomérique non naturelle α par opposition à la configuration anomérique naturelle β. Ces composés chimiques ont fait l'objet d'une demande de brevet EP-A-0 290 583 auquel on pourra se reporter pour la meilleure intelligence de la présente demande.

La demande EP-A-0 290 583, telle que définie à l'article 54(3) CBE, décrit en effet certaines caractéristiques avantageuses de ces composés ainsi que leur procédé de préparation.

Plus précisément, la présente invention concerne une utilisation nouvelle des composés oligonucléotidiques à configuration anomérique α selon la demande de brevet EP-A-0 290 583.

La présente invention a en effet pour objet l'application des composés oligonucléotidiques α constitués par un oligonucléotide ou un oligodésoxynucléotide comportant un enchaînement de nucléotides possédant la configuration anomérique non naturelle α,éventuellement lie de façon covalente à un agent effecteur, tel qu'un agent intercalant, un radical chimique réactif ou photo-activable. Selon la caractéristique essentielle de la présente invention, ces composes sont utilisés en vue de l'inhibition de l'activité des enzymes réverses transcriptases impliquées dans le développement des virus à ARN.

Parmi les composés plus particulièrement concernés par la présente invention, on peut citer les composés oligomères α de formule

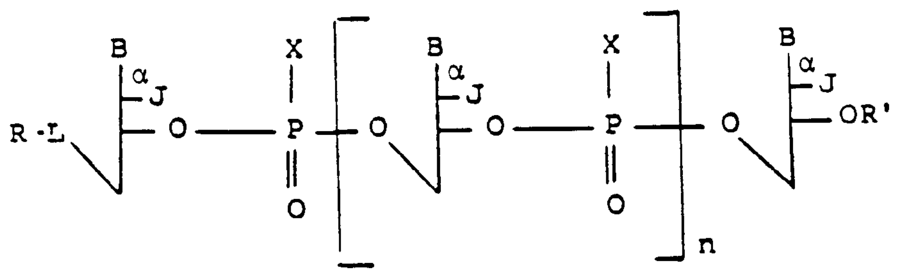

(I)

dans laquelle :

les radicaux B peuvent être identiques ou différents et représentent chacun une base d'un acide nucléique éventuellement modifiée et rattachée au cycle glycosidique selon une configuration anomérique α non naturelle ;

les radicaux X peuvent être identiques ou différents et représentent chacun un oxoanion $O^{\ominus}$, un thioanion $S^{\ominus}$, un groupe alkyle, un groupe alcoxy, aryloxy, un groupe aminoalkyle, un groupe aminoalcoxy, un groupe thioalkyle:

R et R', qui peuvent être identiques ou différents, représentent chacun un atome d'hydrogène ou un groupement -Y-Z ;

Y représente un radical alcoylène droit ou ramifie -alk- ou un radical choisi parmi

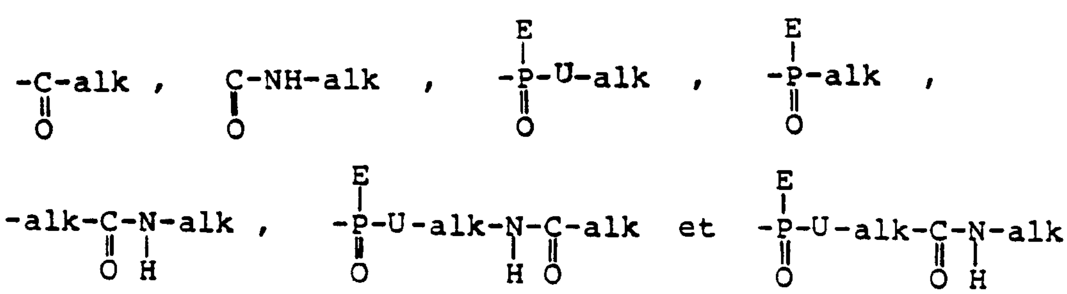

avec U = O, N ou S.

ou bien un radical -Y"-O-Y'- où Y" ou Y' peuvent avoir les significations données pour Y ;

E peut avoir les mêmes significations que X ;

J représente un atome d'hydrogène ou un groupe hydroxy ;

Z est un radical correspondant à un agent effecteur ;

n est un nombre entier y compris O ;

L représente un atome d'oxygène, un atome de soufre ou un groupe -NH-.

Dans cette formule I, on utilise la représentation condensée des nucléosides suivante :

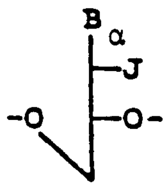

qui correspond à la formule developpée :

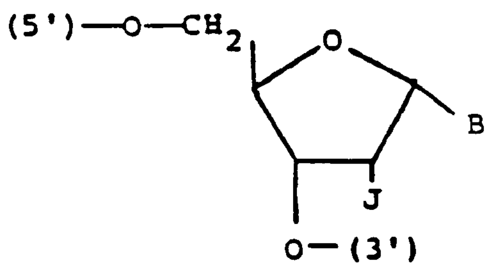

sur laquelle ont été mentionnées les extrémités (3′) et (5′).

Il convient de remarquer que la formule I représente un enchaînement de nucléotides qui peuvent être identiques ou différents, n indiquant simplement le nombre de nucléotides compris dans la molécule ; n est de préférence un nombre compris entre 1 et 50, et de préférence encore entre 1 et 25.

Les radicaux effecteurs correspondent à des agents effecteurs qui sont des composés connus dans les techniques touchant aux acides nucléiques. Il s'agit par exemple des composés capables de "s'intercaler" dans la structure des ADN ou des ARN.

Ces agents d'intercalation sont, en général, constitués par des composés polycycliques ayant une configuration plane tels que l'acridine, la furocoumarine, la daunomycine, la 1,10-phénanthroline, la phénanthridinium, les prophyrines, les dérivés de la dipyrido (1,2-a : 3′, 2′-d) imidazole, l'ellipticine ou l'ellipticinium et leurs dérivés.

Ces agents effecteurs peuvent aussi être des radicaux chimiques réactifs tels que des radicaux chimiques de scission, c'est-à-dire que ces radicaux peuvent directement ou indirectement réagir pour scinder une chaîne de nucléotides. De préférence, ces radicaux chimiques réactifs seront activables, par exemple par voie chimique ou photochimique.

Les groupes réactifs de scission activables sont, par exemple, des dérivés de composés tels que :

l'acide éthylène-diamine-tétracétique,

l'acide diéthylène-triamine-pentaacétique,

les porphyrines,

la 1,10-phénanthroline , le psoralène et autres groupes aromatiques absorbant les radiations du proche U.V. et du visible.

Ces groupements chimiquement activables en présence d'ions métalliques, d'oxygène et d'un ayant réducteur, induisent des coupures dans des séquences d'acides nucléiques situées dans leur voisinage.

Le radical B est constitué par une base d'un acide nucléique liée à la partie sucre du nucléotide par une configuration anomérique α. Ce peut être la thymine, l'adénine, la cytosine, la guanine ou l'uracile. Mais il est également possible d'utiliser des bases d'acides nucléiques modifiées, notamment halogénées ou azidées, par exemple la 5-bromo-uracile ou la 8-azidoadénine ; ou des dérivés aminés comme la 2-amino-adénine et ses dérivés substitués, par exemple sur le $N^6$ par un groupe aminoalkylène ou par un groupe azidophénylalkylène ; ou la guanine substituée sur le $O^6$, par exemple par un groupe (ω-alkylène)-9-acridine ou la 8-(ω-aminoalkyl)-amino-adénine et ses dérivés substitués sur le $NH_2$ en ω par un groupe acridine.

Selon la présente invention, sont donc considérées les bases usuelles, ainsi que la possibilité d'introduction de bases modifiées. Des bases "effectrices", susceptibles de se lier par covalence à un brin β complémentaire, pourront être introduites. Ainsi, la fonctionnalisation de C ou T par un groupement aziridine en position 4 conduit à la formation de pontages covalents $CH_2$ -$CH_2$ entre les deux brins complémentaires sur respectivement G et A.

Donc, plus particulièrement, le radical B est choisi parmi la thymine, l'adénine, la cytosine, la guanine, la 4-azido-cytosine ou la 4-azido-thymine et la 8-azido-adénine, l'uracile, la 5 bromo-uracile.

Le radical X, bien qu'il représente de préférence un oxoanion, peut avoir d'autres significations ; lorsque le radical X représente un groupement alkyle, il s'agit de préférence d'un groupement alkyle inférieur en $C_1$ à $C_7$ et, par exemple, les groupements éthyle, méthyle ou propyle ; lorsque le radical X représente un groupe alcoxy, il s'agit de préférence d'un groupement alcoxy inférieur en $C_1$ à $C_7$, par exemple le groupement méthoxy ou éthoxy ; lorsque X représente un groupement amino-alkyle ou amino-alcoxy, il peut s'agir d'un groupement amino-alkyle mono-substitué, disubstitué ou bien d'un radical amino sous forme de sel d'ammonium quaternaire. Dans ces conditions, les substituants sont, de preférence, des radicaux alkyles inférieurs comme définis précédemment ; quant à la chaîne alkyle ou alcoxy reliant le radical amino au phosphore, il s'agit de préférence d'une chaîne droite ou ramifiée comportant de 1 à 10 atomes de carbone. Lorsque le radical alkyle ou alcoxy est substitué par un hétérocycle azoté, il s'agit notamment de cycle saturé à 5-6 chaînons comportant un atome d'azote qui peut être quaternaire. Enfin, lorsque le radical X est un radical thioalkyle, il s'agit de préférence d'un radical thioalkyle inférieur , c'est-à-dire qui comporte entre 1 et 7 atomes de carbone.

Le radical -alk- est de préférence un radical alcoylène droit ou ramifié ayant de 1 à 10 atomes de carbone.

En particulier, à partir des fonctions alcool terminales 3′ ou 5′ de l'oligomère α, l'effecteur peut donc être introduit via une chaîne $(CH_2)_n$ liée à une fonctionnalisation Z′ de natures diverses à la partie osidique de l'oligomère.

A partir de la formule

$$\begin{matrix}3'\\ \text{ou}\\ 5'\end{matrix}\text{-O-Z'-}(CH_2)_n\text{-effecteur (Z)}$$

on obtiendra, selon ce que représente Z′, par exemple

. un phosphate ou méthyl phosphonate de formule

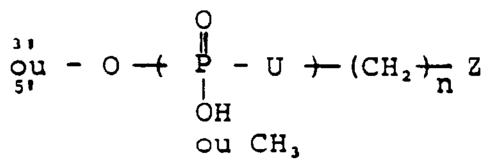

U = O, N ou S

. un éther de formule générale

$$\begin{matrix}3'\\ \text{ou}\\ 5'\end{matrix} - O - CH_2 - (CH_2)_{n-1} - Z$$

. un ester de formule générale

$$-O - CO - (CH_2)_n - Z$$

ou encore

. un carbamate de formule générale

$$-O - CO\ NH - (CH_2)_n - Z$$

Selon la présente invention sont concernés également les composés précédents sous forme de sel avec des bases ou des acides, et les composés sous forme racémique, ou sous forme d'isomères R ou S optiques purifiés ou en mélange.

Selon la présente invention sont concernés également les composés précédents dans les séries D ou L.

De façon très appropriée, l'application des composés oligonucléotidiques α selon la présente invention consiste à inhiber l'activité de l'enzyme réverse transcriptase du virus de l'immunodéficience humaine HIV1

du SIDA.

Le rétrovirus associé à des lymphadénopathies (LAV), également appelé virus HTLV III (HTLV = human T leukemia virus) ou AIDS - related virus (ARV) a été baptisé plus récemment HIV1 et est en effet reconnu comme l'agent responsable du SIDA.

Les virus à ARN et notamment le virus du SIDA ont un mécanisme de multiplication qui fait effectivement intervenir l'enzyme ADN polymérase - ARN dépendante encore appelée transcriptase inverse ou plus communément réverse transcriptase. Cette enzyme copie en ADN monobrin dit complémentaire (ADNc),l'ARN viral. Il faut pour cela qu'elle trouve une amorce oligonucléotidique appariée à l'extrémité 3′ de la matrice d'ARN à copier. L'ARN comporte en général une séquence poly(A) en 3′. Elle ne peut alors synthétiser l'ADNc correspondant dans une réaction exogène qu'à la condition qu'il soit amorcé par un oligo (dT). L'ADNc se synthétise alors par incorporation de dTTP (désoxythymidine 5′ triphosphate) à l'oligo (dT) amorceur.

La transcriptase réverse est à la fois nécessaire à l'établissement de l'état proviral, au démarrage de la réplication du virus, et à l'éventuelle transformation de la cellule.

Bien que le mécanisme d'action des composés oligonucléotidiques $\alpha$ soit encore incomplètement élucidé, il semble qu'ils agissent selon la présente invention en se liant à un site actif de la réverse transcriptase.

Pour ce faire, on utilise de préférence des oligodésoxynucleotides $\alpha$. Il s'agit donc des composés pour lesquels $X = O^{\ominus}$ ; J, R et R′ = H ; B est choisi parmi A, C, T, et G, et L est un atome d'oxygène/dans la formule I.

Parmi ces derniers, on peut encore citer plus particulièrement les homopolydésoxynucléotides $\alpha$, notamment de formule $\alpha dX'_n$ avec X′= A, T, C ou G, et n un nombre entier, de préférence entre 1 et 100, de préférence encore entre 10 et 50. Plus particulièrement X′= T et $n \geqq 10$ ou X′ = C et $n \geqq 30$.

La présente invention a également pour objet l'utilisation des composés oligonucléotidiques $\alpha$ selon l'invention, à la préparation des compositions pharmaceutiques utiles pour le traitement des affections virales des virus à ARN, tels que le virus HIV1 du SIDA, par inhibition de l'activité des enzymes réverses transcriptases impliquées dans les développements des virus à ARN.

D'autres caractéristiques et avantages de la présente invention apparaîtront à la lumière des exemple qui vont suivre.

Dans les exemples qui vont suivre les $\alpha$ oligodésoxynucléotides $\alpha$-dT, $\alpha$-$dT_2$, $\alpha$-$dT_4$, $\alpha$-$dT_6$, $\alpha$-$dT_8$, $\alpha$-$dT_{10}$, $\alpha$-$dT_{12}$, $\alpha$-$dT_{14}$, $\alpha$-$dT_{16}$ ont été dissous dans de l'eau stérilisée pour les essais de réverse transcriptase. Les concentrations ont été déterminées par spectrophotométrie (coefficient d'absorption molaire : 8880, 17760, 35520, 61800, 70000, 83300, 88800, 109800 et 138400, respectivement). Les $\alpha$ oligodésoxynucléotides $\alpha$-$dC_{20}$ et $\alpha$-$dC_{30}$ ont été dissous dans de l'eau distillée et les concentrations ont été déterminées également par spectrophotométrie (coefficient d'absorption molaire 178000 et 267000, respectivement). Les solutions étaient conservées à -20° C jusqu'à leur utilisation.

Le virus HIV à été obtenu à partir de surnageants de culture de lignées cellulaires H9 infectées par HIV (H9/HTLV-IIIb) qui a été fourni par le Docteur R.C. Gallo (National Institutes of Health, Bethesda, Md). Le surnageant a été purifié par centrifugation à basse vitesse et conservé dans des aliquots à -70°C jusqu'à utilisation.

Les réactions de transcription inverse ont été effectuées avec des virus HIV dont partiellement purifiées. Les fluides de culture provenant d'une lignée cellulaire produisant un HUT-78/HTLV-IIIb ont été purifiés par centrifugation à basse vitesse. Des particules virales ont été sédimentées par centrifugation à 100.000 g pendant 30 minutes. Le Pellet viral a été incubé sur glace pendant 30 minutes avec un tampon de solubilisation contenant du triton X-100 0,1 % du dithiothréitol 1mM et du glycérol 50 % et conservé dans des aliquots à -70°C jusqu'à utilisation. Les effets inhibiteurs des $\alpha$ oligodésoxynucléotides contenant des bases thymine sur la réaction de transcription inverse ont été effectués avec une matrice polyrA exogène et un $\alpha$ oligod$T_{12-18}$. Le mélange de réaction (50 $\mu$l) contenait du Tris-HCl 50 mM (pH 7,8), du dithiothréitol 5mM, du glutathione 300 $\mu$M, du EGTA 500 $\mu$M et du KCl 150 mM, du $MgCl_2$ 5 mM, et 1,25 $\mu$g de sérum albumine de bovin, et 1 $\mu$M de (méthyl-$^3$H) dTTP (radioactivité spécifique 30 Ci/mmol.) (5 $\mu$Ci), le mélange comportait également 4,4 $\mu$g/ml de polyrA, 7,5 $\mu$M d'oligodT et du triton X-100 0,03 %, et 10 $\mu$l de solution de composé,(contenant des concentrations variables des composés) et 10 $\mu$l de préparation de réverse transcriptase. Les effets inhibiteurs sur la transcription de HIV à l'aide de $\alpha$-$dC_{20}$ et $\alpha$-$dC_{30}$ ont été déterminés en utilisant une matrice exogène polyl (50 $\mu$g/ml) tandis que le mélange oligod$C_{12-18}$ servait d'amorce (0,44 U/ml). Le mélange de réaction avec le système polyl.oligodC contenait des réactifs décrits pour le système polyrA. oligodT mais les concentrations de MgCl et KCl ont été ajustées à 2,5 mM et 200 $\mu$M, respectivement, tandis que le dTTP trifié a été substitué par le (méthyl-$^3$H)dCTP (radioactivité spécifique 19,5 Ci/mmol) (5 $\mu$Ci). Les mélanges de réactions ont été incubés à 37°C pendant 0 à 60 minutes à quel moment 100 $\mu$l d'ADN de thymus de veau (150 $\mu$g/ml), 2 ml de $Na_4P_2O_7$ (0,1 M dans HCL 1 N) et 2 ml de TCA (10 % v/v) ont été ajoutés. Les solutions ont été gardées sur glace pendant au moins 30 minutes, après lesquelles la matière acide insoluble a été lavée et analysée quant

à sa radioactivité.

EXEMPLE 1 - Inhibition in vitro de la transcriptase réverse du virus de l'immunodéficience humaine HIV1 à l'aide de l'homopolydésoxynucléotide $\alpha dT_{15}$

Ont été étudiés :
a) l'aptitude de l'oligodésoxynucléotide α $\alpha[d(Tp)_{14}T]$ à servir d'amorce dans la réaction de transcription inverse à partir de la réverse transcriptase de HIV1 partiellement purifiée et d'une matrice poly(rA), puis
b) l'effet inhibiteur de ce même oligodésoxynucléotide α ($\alpha dT_{15}$) sur la réaction de transcription inverse du système matrice-amorce : poly(rA).$\beta d[(Tp)_9T]$
a) Détermination de l'aptitude de $\alpha dT_{15}$ à amorcer la réaction exogène de transcription inverse de HIV1.
L'enzyme réverse transcriptase utilisée provenait de cellules MT infectées avec la souche. HTLV-IIIB, elle était purifiée partiellement par ultracentrifugation.

Les valeurs du tableau I ci-dessous indiquent une incorporation de méthyl $^3H$ dTTP (22052 Cpm), lors de la réaction de transcription inverse sur le système matrice-amorce : poly(rA).$\beta d[(Tp)_9T]$, par contre il n'y a pas d'incorporation de méthyl $^3H$ dTTP lorsque l'amorce $\beta d[(Tp)_9T]$ est remplacée par l'oligonucléotide $\alpha d[(Tp)_{14}T]$ à des concentrations variant de 5 à 320 µMolaire. Ces résultats indiquent que l'oligonucléotide $\alpha d[(Tp)_{14}T]$ ne peut pas fonctionner comme amorce pour la réaction de transcription inverse. La radioactivité indique la quantité d'incorporation de méthyl $^3H$ dTTP et a été mesurée essentiellement comme décrit antérieurement dans Proc. Natl. Acad. Sci. USA 72, 284-288 Declerq et à. (1975).

TABLEAU I

| | $\alpha dT_{15}$ Concentration µMole | Cpm (valeur moyenne) | % inhibition |
|---|---|---|---|
| Contrôle (1) | 0 | 22052 | 0 |
| Composé $\alpha dT_{15}$ | 0 | 1204 | 95 |
| | 5 | 374 | 98 |
| | 20 | 1472 | 93 |
| | 80 | 629 | 97 |
| | 320 | 1098 | 95 |

(1) le mélange de contrôle exogène contenait 7,5 µM d'oligo $\alpha dT_{10}$.

b) Effets inhibiteurs de l'oligonucléotide $\alpha dT_{15}$. sur la réaction de transcription inverse du système matrice-amorce poly(rA).$d((Tp)_9T]$.

Les valeurs reproduites dans le tableau II suivant indiquent que $\alpha dT_{15}$ rentre en compétition avec l'amorce α $oligo(dT)_{10}$ pour la réaction exogène de transcription inverse de HIV1.

TABLEAU II

| | Concentration µMole en $\alpha dT_{15}$ | Cpm (valeur moyenne) | % inhibition |
|---|---|---|---|
| Contrôle (1) | 0 | 22052 | 0 |
| Composé $\alpha dT_{15}$ (1) + Contrôle | 5 | 2360 | 89 |
| | 20 | 1945 | 91 |
| | 80 | 2441 | 89 |
| | 320 | 1528 | 93 |

(1) le mélange de réaction exogène contenait 7,5 µM d'oligo $\beta dT_{10}$.

En effet, ces valeurs attestent que lorsque l'oligo-nucléotide $\alpha d[(Tp)_{14}T]$ est ajouté dans des concentrations variant de 5 à 320 µM au mélange de la réaction de transcription inverse sur le système poly(rA).β-d $[(Tp)_9T]$ on observe une inhibition quantitative de l'incorporation du méthyl $^3H$ dTTP.

Par conséquent, l'oligonucléotide $\alpha d[(Tp)_{14}T]$ qui est complémentaire de la matrice poly(rA) inhibe de façon efficace la réaction de transcription inverse.

EXEMPLE 2 - Inhibition in vitro de la transcriptase réverse du virus de l'immunodéficience humaine HIV1 du SIDA à l'aide de l'homopolydésoxynucléotide $\alpha dT_n$

Une série d'oligodésoxynucléotides ont été synthétises contenant des bases thymidines liées à des cycles désoxyriboses en configuration anomérique α toujours selon le procédé décrit dans le brevet 87 04 339 .

Le nombre de monomères variait de 1 à 16. Il s'agissait plus particulièrement de $\alpha dT$, $\alpha dT_2$, $\alpha dT_4$, $\alpha dT_6$, $\alpha dT_8$, $\alpha dT_{10}$, $\alpha dT_{12}$, $\alpha dT_{14}$, $\alpha dT_{15}$, $\alpha dT_{16}$.

La réverse transcriptase de HIV1 ci-après abrégé RT a été partiellement purifiée et les oligomères α ont été étudiés en fonction de leur faculté à servir d'amorce sur une matrice poly(A) dans une réaction de transcription inverse exogène.

En l'absence de l'amorce, ici $\beta d(T)_{12-18}$ (mélange d'hormopolydésoxynucléotides de 12 à 18 monomères), aucune incorporation de (méthyl-$^3H$) dTTP n'a pu être détectée même à des concentration en oligomère $\alpha dT$ jusqu'à 100 µM. Ce résultat indique que ces oligomères α ne fonctionnent pas comme amorce pour la réaction de transcription inverse de HIV1.

Toutefois, quand ces composés ont été ajoutés au mélange de réaction de transcription inverse en présence d'amorce $\beta dT_{12-18}$, une inhibition de l'ordre de 50% de la réaction de transcription inverse a pu être observée avec des concentrations de l'ordre de 1 µM (ED 50) avec $\alpha dT_{10}$, $\alpha dT_{12}$, $\alpha dT_{14}$, $\alpha dT_{15}$, $\alpha dT_{16}$, et même une inhibition complète ou quasi complète pour des concentrations de 4 µM de $\alpha dT_n$ $n > 10$, tandis qu'aucune inhibition significative n'a pu être observée avec $\alpha dT$, $\alpha dT_2$, $\alpha dT_4$ et $\alpha dT_6$.

Le tableau III ci-après reproduit les pourcentages d'inhibitions de la réaction exogène de transcription inverse avec différents oligomères $\alpha dT_n$.

TABLEAU III

| Concentr.(uM) | dT | dT2 | dT4 | dT6 | dT10 | dT12 | dT14 | dT15 | dT16 |
|---|---|---|---|---|---|---|---|---|---|
| 0 | 0 | 0 | 0 | 0 | 0 | 0 | 0 | 0 | 0 |
| 0.16 | 22 | 21 | 17 | 19 | -10 | 16 | 6 | 19 | 12 |
| 0.8 | 8 | 22 | 15 | 7 | 1 | 34 | 23 | 23 | 36 |
| 4 | 19 | 26 | -13 | 15 | 45 | 86 | 98 | 98 | 98 |
| 20 | 46 | 5 | 27 | 0 | 71 | 97 | 99 | 99 | 99 |
| 100 | 23 | 23 | 30 | 6 | 97 | 99 | 99 | 100 | 100 |
| $ED_{50}$* | >100 | >100 | >100 | >100 | 5.5 | 1.3 | 1.4 | 1.4 | 1.2 |

* dose requise pour inhiber la réaction de transcription inverse de 50 %.

Ces résultats indiquent également que les oligomères $\alpha dT_n$ qui sont complémentaires de la matrice βpoly(A) bloquent de manière efficace la réaction de transcription inverse de HIV1 pour n ≧ à 10.

Ces pourcentages ont été calculés de la même manière que précédemment par mesure de radioactivité, laquelle indique la quantité d'incorporation de méthyl $^3H$ dTTP (valeurs en Cpm).

Pour $\alpha dT_{16}$, ces valeurs sont reproduites à titre illustratif au tableau IV suivant.

TABLEAU IV

| | Conc. $\alpha dT_{16}$ (µM) (2) | Cpm | % inhibition |
|---|---|---|---|
| Contrôle (1) | 0 | 286988 | 0 |
| Composé $\alpha dT_{16}$ + Contrôle (1) | 0.16 | 251971 | 12 |
| | 0.8 | 183491 | 36 |
| | 4 | 5718 | 98 |
| | 20 | 2922 | 99 |
| | 100 | 1216 | 100 |

1) Ce mélange exogène contenait 7,5 µM d'oligo$(dT)_{12-18}$.

2) Les concentrations en $\alpha dT_{16}$ ont été déterminées par spectrophotométrie.

EXEMPLE 3 - Inhibition in vitro de la transcriptase réverse du virus de l'immunodéficience humaine HIV1 du SIDA à l'aide de l'homopolydésoxynucléotide $\alpha dC_n$

Sont également testés ci-après les composés $\alpha dC_n$. Alors que $E_{D50}$ (dose requise pour une inhibition de

50 % de la réaction de transcription inverse) était supérieure à 100 μM pour $\alpha dC_{20}$, des résultats tout à fait intéressants ont été obtenus avec $dC_{30}$ pour lequel $E_{D50}$ était de 29 μM mais en utilisant comme matrice exogène du polyI et de l'oligo $dC_{12-18}$ comme amorce.

EXEMPLE 4 - Effets inhibiteurs des composés sur la proliferation de cellules de tumeur humaine et murine.

Les données du tableau V suivant montrent que les nucléosides α constitutifs n'ont pas d'activité biologique et ne,sont pas cytotoxiques pour les cellules L1210/0, L1210/BdUrd (résistant à BdUrd), CEM, MT4, MOLT/4F, contrairement à certains β désoxynucléosides.

TABLEAU V

| Composé | $ID_{50}$[a] (μM) | | | | |
|---|---|---|---|---|---|
| | L1210/0 | L1210/BdUrd | CEM | MT4 | MOLT/4F |
| α-2'-dGuo | > 500 | > 500 | > 500 | > 500 | > 500 |
| α-2'-dAdo | > 500 | > 500 | > 500 | > 500 | > 500 |
| α-2'-dThd | > 500 | > 500 | > 500 | > 500 | > 500 |
| α-2'-dC | > 500 | > 500 | > 500 | > 500 | > 500 |
| β dGuo | 58.2 ± 10.9 | 55.5 ± 6.8 | 22.1 ± 0.6 | 55.3 ± 5.3 | 79.5 ± 9.2 |
| β dAdo | > 500 | > 500 | 189 ± 19 | > 500 | > 500 |
| β dThd | 29.0 ± 1.9 | > 500 | 24.7 ± 3.42 | 191 ± 7 | > 500 |

a : dose d'inhibition du développement cellulaire de 50 % moyenne pour au moins deux expériences différentes.

## Revendications

1. Utilisation des composés constitués par un oligonucléotide ou un oligodésoxynucléotide comportant un enchaînement de nucléotides possédant la configuration anomérique non naturelle α, éventuellement lié de façon covalente à un agent effecteur, pour l'obtention d'une composition utile pour le traitement des affections virales des virus à ARN par inhibition de l'activité des enzymes réverses transcriptases impliquées dans les développements des virus à ARN.

2. Utilisation selon la revendication 1, caractérisée en ce que les composés ont pour formule

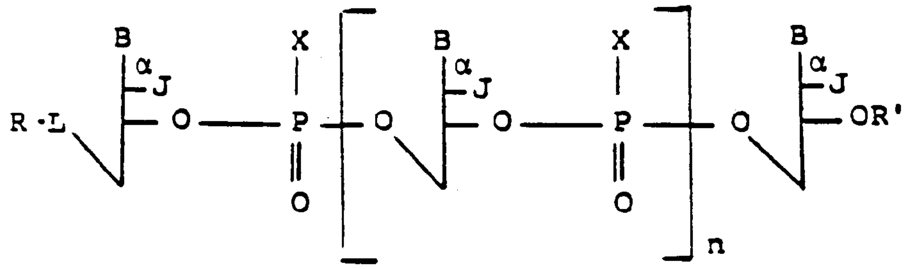

(I)

dans laquelle :

les radicaux B peuvent être identiques ou différents et représentent chacun une base d'un acide nucléique éventuellement modifiée et rattachée au cycle glycosidique selon une configuration anomérique α non naturelle ;

les radicaux X peuvent être identiques ou différents et représentent chacun un oxoanion $O^{\ominus}$, un thioanion $S^{\ominus}$, un groupe alkyle, un groupe alcoxy, aryloxy, un groupe aminoalkyle, un groupe aminoalcoxy, un groupe thioalkyle ;

R et R′, qui peuvent être identiques ou différents, représentent chacun un atome d'hydrogène ou un groupement -Y-Z ;

Y représente un radical alcoylène droit ou ramifié -alk- ou un radical choisi parmi

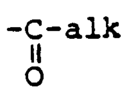 , 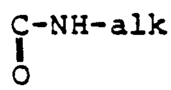 , 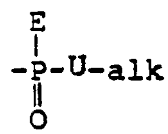 , 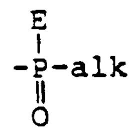 ,

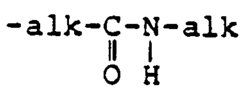 , 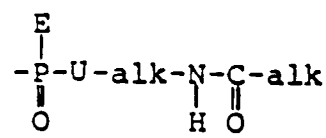 et 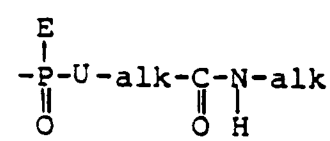

avec U = O, N ou S.

ou bien un radical -Y″-O-Y′- où Y″ ou Y′ peuvent avoir les significations données pour Y ;

E peut avoir les mêmes significations que X ;

J représente un atome d'hydrogène ou un groupe hydroxy ;

Z est un radical correspondant à un agent effecteur ;

n est un nombre entier y compris O ;

L représente un atome d'oxygène, un atome de soufre ou un groupe -NH-.

3. Utilisation selon l'une des revendications précédentes, dans laquelle le virus à ARN est le virus de l'immunodéficience humaine HIV1 du SIDA.

4. Utilisation selon l'une des revendications 2 ou 3, caractérisée en ce que les composés sont des oligodésoxynucléotides α pour lesquels $X = O^{\ominus}$ ; J, R, R′ = H : B est choisi parmi A, C, T et G ; et L est un atome d'oxygène dans la formule I.

5. Utilisation selon la revendication précédente, caractérisée en ce que les composés sont des homopolydésoxynucléotides α.

6. Utilisation selon la revendication précédente, caractérisée en ce que les composés ont pour formule $\alpha dX'_n$, avec X′ = A, C, T ou G et n est un nombre entier, de préférence compris entre 1 et 100.

7. Utilisation selon la revendication précédente, caractérisée en ce que n est compris entre 10 et 50.

8. Utilisation selon l'une des revendications 6 ou 7, caractérisée en ce que X′ = T et n ≧ 10.

9. Utilisation selon l'une des revendications 6 ou 7, caractérisée en ce que X′ = C et n ≧ 30.

## Claims

1. Use of compounds consisting of an oligonucleotide or an oligodeoxynucleotide having a nucleotide chain possessing the non-natural anomeric α-configuration, possibly covalently bound to an effector agent, for obtaining a composition useful for the treatment of viral infections of RNA viruses in inhibition the activity of reverse transcritase enzymes involved in the development of RNA viruses.

2. Use according to Claim 1, characterized in that the compounds have the formula:

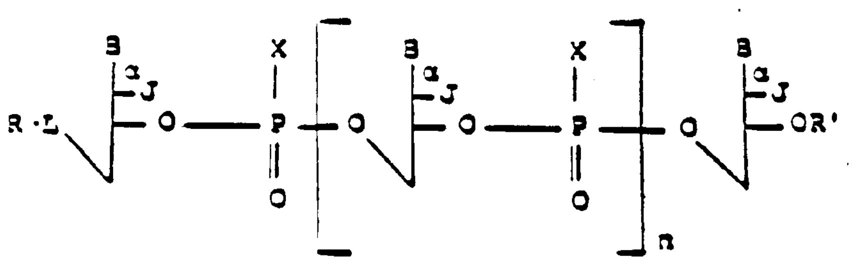 (I)

in which:

the radicals B can be identical or different and each represent a base of a nucleic acid, which may be modified and attached to the glycoside ring in a nonnatural anomeric α-configuration;

the radicals X can be identical or different and each represent an oxygen anion $O^{\ominus}$, a sulfur anion $S^{\ominus}$, an alkyl group, an alkoxy or aryloxy group, an aminoalkyl group, an aminoalkoxy group, or a thioalkyl group;

R and R′, which can be identical or different, each represent a hydrogen atom or a -Y-Z group;

Y represents a straight-chain or branched alkylene radical -alk- or a radical chosen from among

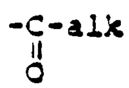 , 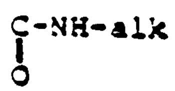 , 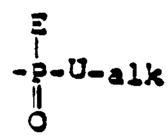 , 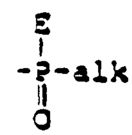 ,

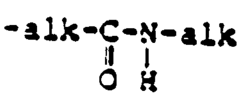 , 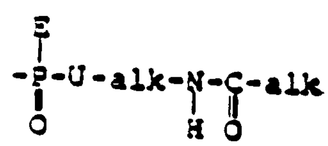 and 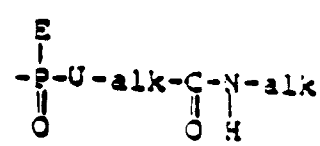

with U = O, N or S,

or else a radical -Y″-O-Y′- where Y″ or Y′ can have the meanings given for Y;

E can have the same meanings as X;

J represents a hydrogen atom or a hydroxyl group;

Z is a radical corresponding to an effector agent;

n is an integer, including 0;

L represents an oxygen atom, a sulfur atom or an -NH- group.

3. Use, according to one of the preceding claims, wherein the RNA virus is the AID human immunodeficiency virus HIV1.

4. Use according to one of Claims 2 or 3, characterized in that the compounds are α-oligodeoxynucleotides for which $X = O^{\ominus}$; J, R, R′ = H; B is chosen from among A, C, T and G; and L is an oxygen atom in formula I.

5. Use according to the preceding claim, characterized in that the compounds are α-homopolydeoxynucleotides.

6. Use according to the preceding claim, characterised in that the compounds have the formula $\alpha dX_n'$, with $X'$ = A, C, T or G and n an integer preferably between 1 and 100.

7. Use according to the preceding claim, characterized in that n is between 10 and 50.

8. Use according to one of Claims 6 or 7, characterized in that $X'$ = T and $n \geqq 10$.

9. Use according to one of Claims 6 or 7, characterized in that $X'$ = C and $n \geqq 30$.

**Patentansprüche**

1. Verwendung von Verbindungen, die bestehen aus einem Oligonucleotid oder einem Oligodesoxynucleotid, das eine Verknüpfung von Nucleotiden aufweist, welche die nicht-natürliche α-anomere Konfiguration aufweisen und gegebenenfalls kovalent an ein Effektor-Agens gebunden sind, zur Herstellung einer Zusammensetzung, die verwendbar ist für die Behandlung von Virenerkrankungen, hervorgerufen durch RNA-Viren, durch Inhibierung der Aktivität der Revers-Transkriptase-Enzyme, die an der Entwicklung der RNA-Viren beteiligt sind.

2. Verwendung nach Anspruch 1, dadurch gekennzeichnet, daß die Verbindungen die Formel haben

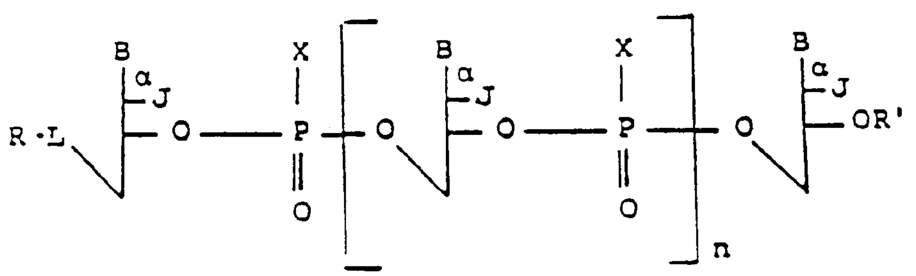

(I)

in der bedeuten:
die Reste B, die identisch oder verschieden sein können, jeweils eine Base einer Nucleinsäure, die gegebenenfalls modifiziert ist und in einer nicht-natürlichen α-anomeren Konfiguration an den Glycosid-Cyclus gebunden ist;
die Reste X, die identisch oder verschieden sein können, jeweils ein Oxoanion $O^{\ominus}$, ein Thioanion $S^{\ominus}$, eine Alkylgruppe, eine Alkoxygruppe, eine Aryloxygruppe, eine Aminoalkylgruppe, eine Aminoalkoxygruppe, eine Thioalkylgruppe;
R und R′, die identisch oder verschieden sein können, jeweils ein Wasserstoffatom oder eine Gruppe -Y-Z;
Y einen geraden (unverzweigten) oder verzweigten Alkylenrest -alk- oder einen Rest, ausgewählt aus

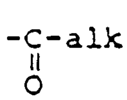 , 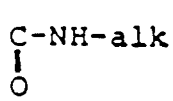 , 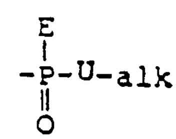 , 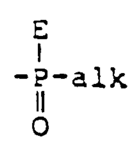 ,

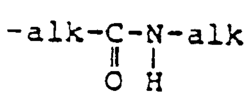 , 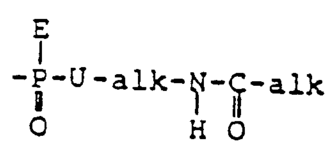 und 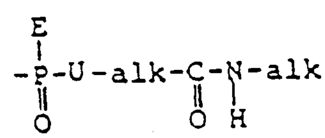

mit U = O, N oder S, oder
auch einen Rest -Y″-O-Y′-, worin Y″ oder Y′ die oben für Y angegebenen Bedeutungen haben können;
E die gleichen Bedeutungen wie X haben kann;
J ein Wasserstoffatom oder eine Hydroxygruppe;

Z einen Rest, der einem Effektor-Agens entspricht;
n eine ganze Zahl y einschließlich 0;
L ein Wasserstoffatom, ein Schwefelatom oder eine -NH-Gruppe.

**3.** Verwendung nach einem der vorhergehenden Ansprüche, bei der der RNA-Virus der Human-Immundefekt-Virus HIV1 von AIDS ist.

**4.** Verwendung nach einem der Ansprüche 2 oder 3, dadurch gekennzeichnet, daß die Verbindungen α-Oligodesoxynucleotide sind, bei denen in der Formel I bedeuten $X = O^{\ominus}$; J, R, R′ = H; B ausgewählt wird aus A, C, T und G; und L ein Sauerstoffatom ist.

**5.** Verwendung nach dem vorhergehenden Anspruch, dadurch gekennzeichnet, daß die Verbindungen α-Homopolydesoxynucleotide sind.

**6.** Verwendung nach dem vorhergehenden Anspruch, dadurch gekennzeichnet, daß die Verbindungen die Formel $\alpha dX'_n$ haben mit X′ = A, C, T oder G und n = eine ganze Zahl, vorzugsweise zwischen 1 und 100.

**7.** Verwendung nach dem vorhergehenden Anspruch, dadurch gekennzeichnet, daß n zwischen 10 und 50 liegt.

**8.** Verwendung nach einem der Ansprüche 6 oder 7, dadurch gekennzeichnet, daß X′ = T und $n \geqq 10$.

**9.** Verwendung nach einem der Ansprüche 6 oder 7, dadurch gekennzeichnet, daß X′ = C und $n \geqq 30$.